# EUROPÄISCHE PATENTSCHRIFT

(11) **EP 0 741 703 B1**
(45) Veröffentlichungstag und Bekanntmachung des Hinweises auf die Patenterteilung: **11.04.2001**
(21) Anmeldenummer: 95906982.4
(22) Anmeldetag: 20.01.1995
(51) Int. Cl.: C07D 211/46, C07D 211/58, C08K 5/3435

(54) **VERFAHREN ZUR HERSTELLUNG VON N,N'-VERBRÜCKTEN BIS-TETRAMETHYLPIPERIDINYL-VERBINDUNGEN**
PROCESS FOR PRODUCING N,N'-BRIDGED BIS-TETRAMETHYL PIPERIDINYL COMPOUNDS
PROCEDE DE PREPARATION DE COMPOSES BIS-TETRAMETHYLPIPERIDINYLE A PONTS N, N'

(30) Priorität: 02.02.1994 DE 4403085
(43) Veröffentlichungstag der Anmeldung: 13.11.1996
(73) Patentinhaber: BASF AKTIENGESELLSCHAFT, 67056 Ludwigshafen (DE)
(72) Erfinder: AUMÜLLER, Alexander, D-67435 Neustadt (DE)
(86) Internationale Anmeldenummer: EP9500205
(87) Internationale Veröffentlichungsnummer: WO9521157

(56) Entgegenhaltungen:
- DE-A- 2 338 076
- PATENT ABSTRACTS OF JAPAN vol. 11, no. 276 (P-613) (2723) 8. September 1987 & JP,A,62 075 526 (KONISHIROKU) 7. April 1987
- INFORM. CHIM., Bd.111, 1972 Seiten 133 - 137 HECHLER, G. 'Ethylene and Propylene Carbonates'
- LAKOKRAS. MATER. IKH PRIMEN, Bd.6, 1985 Seiten 27 - 30 MIKHEEV, V. V. ET. AL.

## Beschreibung

Die vorliegende Erfindung betrifft ein verbessertes Verfahren zur Herstellung von N,N'-verbrückten Bis-tetramethylpiperidinyl-Verbindungen der allgemeinen Formel I 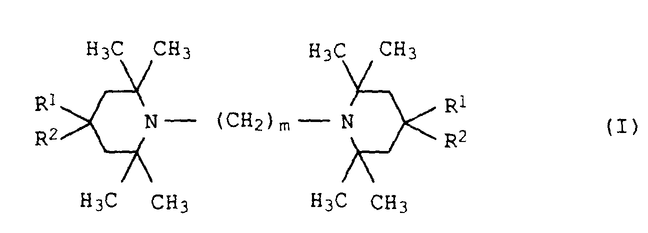 in der die Variable m für die Zahl 2 oder 3 steht und die beiden Restepaare R¹/R² gleich oder verschieden sind und folgende Bedeutungen haben können:
(a)
   - R¹: Wasserstoff und R² eine Gruppierung der Formel -A-R³, wobei
   - A: Sauerstoff oder ein Brückenglied der Formel -NR⁴-, -NR⁵-CO-, -O-CO-, -O-CO-NR⁵- oder O-CO-O- bedeutet,
   - R³: Wasserstoff, C₁- bis C₂₀-Alkyl, das durch bis zu 10 nicht benachbarte Sauerstoffatome oder Brückenglieder der Formel -NR⁴- und durch bis zu 3 Carbonylgruppen unterbrochen sein kann, C₂- bis C₂₀-Alkenyl, C₃- bis C₁₂-Cycloalkyl, das durch bis zu 3 C₁- bis C₄-Alkylgruppen substituiert sein kann, Phenyl, das durch bis zu 3 C₁- bis C₆-Alkylgruppen oder Hydroxylgruppen substituiert sein kann, C₇- bis C₁₈-Phenylalkyl, das durch bis zu 3 C₁- bis C₄-Alkylgruppen oder Hydroxylgruppen substituiert sein kann, C₆- bis C₁₈-Cycloalkyl-alkyl, das durch bis zu 3 C₁- bis C₄-Alkylgruppen substituiert sein kann, oder einen fünf- oder sechsgliedrigen ungesättigten oder gesättigten heterocyclischen Ring mit bis zu 3 Heteroatomen aus der Gruppe Stickstoff, Sauerstoff und Schwefel, welcher zusätzlich benzanelliert und durch bis zu 3 C₁- bis C₆-Alkylgruppen oder Hydroxylgruppen substituiert sein kann, bezeichnet, mit der Maßgabe, daß R³ nicht Wasserstoff bezeichnet, falls A das Brückenglied -NR⁴- bedeutet,
   - R⁴: C₁- bis C₂₀-Alkyl, C₂- bis C₂₀-Alkenyl, C₃- bis C₁₂-Cycloalkyl, das durch bis zu 3 C₁- bis C₄-Alkylgruppen substituiert sein kann, C₇- bis C₁₈-Phenylalkyl, das durch bis zu 3 C₁- bis C₄-Alkylgruppen substituiert sein kann, oder C₆- bis C₁₈-Cycloalkyl-alkyl, das durch bis zu 3 C₁- bis C₄-Alkylgruppen substituiert sein kann, bedeutet und
   - R⁵: Wasserstoff oder C₁- bis C₄-Alkyl bezeichnet;
(b)
   - R¹: Wasserstoff und R² C₁- bis C₆-Alkoxycarbonyl-C₁- bis C₂₀-alkyloxy oder C₁- bis C₆-Alkoxycarbonyl-C₂- bis C₂₀-alkenyloxy;
(c)
   - R¹ und R²: Gruppierungen der Formel -O-R⁶, wobei die beiden Reste R⁶ jeweils gleich oder verschieden sind, für C₁- bis C₂₀-Alkyl oder C₂- bis C₂₀-Alkenyl, welches durch bis zu 3 Hydroxylgruppen substituiert sein kann, stehen und unter Ausbildung eines 1,3-Dioxan- oder eines 1,3-Dioxolan-Ringes verknüpft sein können;
(d)
   - R¹ / R²: das Carbonyl-Sauerstoffatom eines Piperidon-Ringes, wobei diese Sauerstoffatome auch unter Ausbildung der entsprechenden Enol-Struktur am Piperidon-Ring durch Gruppierungen der Struktur R⁴ verethert sein können;
ausgehend von Tetramethylpiperidinyl-Verbindungen der allgemeinen Formel II

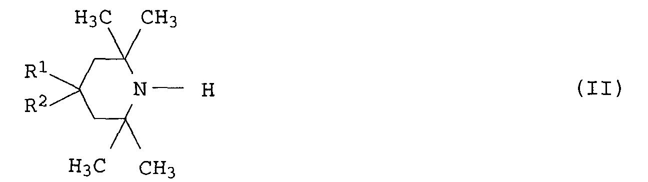

Die Erfindung betrifft außerdem ein Verfahren zur Herstellung von N,N'-verbrückten Bis-tetramethylpiperidinylestern.

Verfahren zur Herstellung von N-ethylenverbrückten Tetraalkylpiperidinen sind bekannt. So beschreiben L.M. Kostochka, A.M. Belostotskii und A.P. Skoldinov in den Literaturstellen Khim. Geterots. Soedinenii 1981, 1694-95 (1) und Khim. Geterots. Soedinenii 1982, 1657-61 (2) die photochemische Dimerisierung von 1,2,2,6,6-Pentamethylpiperidin-4-ol zu N,N'-ethylenverbrücktem Bis-2,2,6,6-tetramethylpiperidin-4-ol (Verbindung I mit m = 2, R¹ = H und R² = OH). Neben einer unbefriedigenden Ausbeute besitzt diese Methode jedoch noch weitere Nachteile. So entstehen zahlreiche Nebenprodukte, die eine aufwendige Reinigung erfordern. Die Belichtung ist technisch aufwendig und damit unwirtschaftlich. Zur Herstellung des verbrückten Produktes muß aus dem technisch gut zugänglichen 2,2,6,6-Tetramethylpiperidin-4-ol erst das N-methylierte Derivat als zusätzliche Stufe hergestellt werden.

In Beispiel 1 der DE-OS 23 38 076 (3) wird die Herstellung von 1,2-Bis(4-benzoyloxy-2,2,6,6-tetramethylpiperidino)-ethan durch Umsetzung des N-unsubstituierten 4-Benzoyloxy-2,2,6,6-tetramethylpiperidins mit 1,2-Dibromethan beschrieben. Diese Verfahrensweise hat den gravierenden Nachteil, daß das hochtoxische 1,2-Dibromethan als Reagenz verwendet wird. Auch die anschließende aufwendige Aufarbeitung der Reaktionsmischung durch Extraktion mit (dem ebenfalls toxischen) Benzol, Waschen, Eindampfen, erneutes Waschen und Umkristallisation läßt vermuten, daß diese Reaktion ebenfalls nicht selektiv zum gewünschten Produkt abläuft. Die in (3) genannten verbrückten Piperidinderivate werden als Stabilisatoren für synthetische Polymere gegen Licht- und thermischen Abbau empfohlen.

Aufgabe der vorliegenden Erfindung war es daher, ein Verfahren bereitzustellen, das in hoher Ausbeute und Selektivität unter Vermeidung des Einsatzes toxischer Halogenalkane einen wirtschaftlich günstigen Zugang zu N-ethylen- oder N-propylenverbrückten Tetramethylpiperidin-Verbindungen ermöglicht.

Demgemäß wurde ein Verfahren zur Herstellung der eingangs definierten N,N'-verbrückten Bis-tetramethylpiperidin-Verbindungen I ausgehend von den eingangs genannten Tetramethylpiperidin-Verbindungen II gefunden, welches dadurch gekennzeichnet ist, daß man die Verbindungen II mit einem cyclischen Carbonat der allgemeinen Formel III 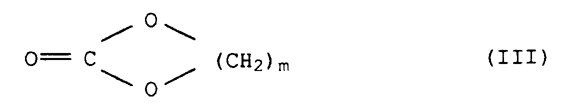 umsetzt.

Die beiden Restepaare R¹/R² sind vorzugsweise gleich. Sollen jedoch Verbindungen I mit verschiedenen Substituenten an den 4-Positionen der beiden Piperidinylringe hergestellt werden, geht man zweckmäßigerweise von Mischungen der Ausgangsverbindungen II aus.

Die Brückenglieder A bei Bedeutung (a) sind vorzugsweise so eingebaut, daß das jeweilige Sauerstoffatom oder Stickstoffatom direkt an den Piperidinylring gebunden ist. Bei der Urethan-Gruppierung -O-CO-NR⁵- sind O- und N-Substitution gleichermaßen möglich. Verbindungen I mit direkt an die 4-Position des Piperidinylringes gebundener Carbonylfunktion können ebenfalls nach dem erfindungsgemäßen Verfahren hergestellt werden, jedoch sind die entsprechenden Ausgangsverbindungen II schwieriger als im Fall der O- oder N-Substitution zu synthetisieren.

Die vorliegende Erfindung betrifft außerdem ein Verfahren zur Herstellung von N,N'-verbrückten Bis-tetramethylpiperidinylestern der allgemeinen Formel Ib 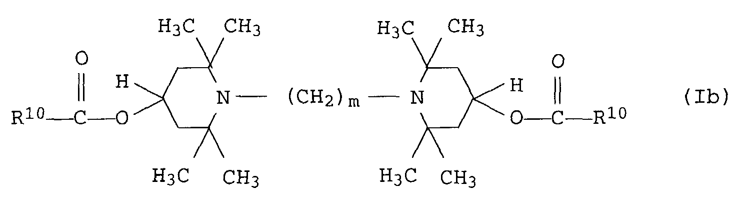 in der die Variable m für die Zahl 2 oder 3 steht und R¹⁰ C₁- bis C₂₀-Alkyl oder C₂- bis C₂₀-Alkenyl, welches durch bis zu 3 Hydroxylgruppen substituiert sein kann, C₁- bis C₂₀-Alkoxy, C₃- bis C₁₂-Cycloalkyl, das durch bis zu 3 C₁- bis C₄-Alkylgruppen substituiert sein kann, Phenyl, das durch bis zu 3 C₁- bis C₆-Alkylgruppen oder Hydroxylgruppen substituiert sein kann, oder C₇- bis C₁₈-Phenylalkyl, das durch bis zu 3 C₁- bis C₄-Alkylgruppen oder Hydroxylgruppen substituiert sein kann, bedeutet, welches dadurch gekennzeichnet ist, daß man nach dem erfindungsgemäßen Verfahren hergestellte N,N'-überbrückte Bis-tetramethyl-hydroxypiperidinyl-Verbindungen der Formel Ic 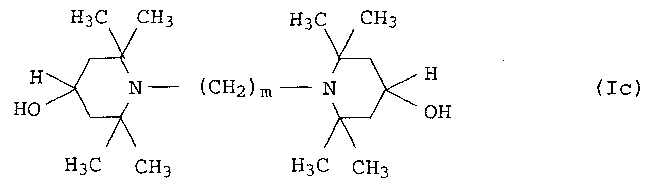 mit Carbonsäurederivaten der allgemeinen Formel R¹⁰-CO-R¹¹ umsetzt, in der R¹¹ Chlor, Brom, C₁- bis C₄-Alkoxy oder eine Gruppe der Formel -O-CO-R¹⁰ bezeichnet.

Als geradkettige oder verzweigte Alkylreste für R³ bis R⁶ und R¹⁰ sowie in R² bei Bedeutung (b) und als Substituenten an cycloaliphatischen Ringen, an aromatischen Ringen und an heterocyclischen Ringen, die als C₁- bis C₄-, C₁- bis C₆- und C₁- bis C₂₀-Alkylreste angesprochen sind, eignen sich beispielsweise Methyl, Ethyl, n-Propyl, iso-Propyl, n-Butyl, iso-Butyl, sec.-Butyl, tert.-Butyl, n-Amyl, iso-Amyl, sec.-Amyl, tert.-Amyl, Neopentyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Pentadecyl, n-Hexadecyl, n-Heptadecyl, n-Octadecyl, n-Nonadecyl und n-Eicosyl. Bevorzugt werden hiervon, insbesondere als Substituenten an cycloaliphatischen, aromatischen und heterocyclischen Ringen, C₁- bis C₄-Alkylreste, insbesondere Methyl und Ethyl. Als Alkylreste können auch technische oder natürlich vorkommende Gemische verschiedener derartiger Reste auftreten.

Durch nicht benachbarte Sauerstoffatome, Brückenglieder -NR⁴- und Carbonylgruppen unterbrochene Alkylreste für R³ sind beispielsweise Gruppierungen der Formel -(CH₂CH₂O)ₙ-CH₂CH₃ (n = 1 bis 9), -CH₂CH₂-N(CH₃) -CH₂CH₃, -CH₂CH₂-N(CH₃) -CH₂CH₂CH₃ und -CH₂CH₂-CO-CH₃.

Als geradkettige oder verzweigte C₂- bis C₂₀-Alkenylreste für R³, R⁴, R⁶ und R¹⁰ sowie in R² bei Bedeutung (b) eignen sich z.B. Vinyl, Allyl, Methallyl, 1-Propenyl, 4-Pentenyl, Oleyl, Linolyl oder Linolenyl. Bevorzugt werden hierbei C₂- bis C₅- und C₁₆- bis C₁₈-Alkenylreste.

Als geradkettige oder verzweigte C₁- bis C₂₀-Alkoxyreste für R¹⁰ kommen insbesondere C₁- bis C₄-Alkoxyreste in Betracht. Unter den C₁- bis C₄-Alkoxyresten für R¹⁰ und R¹¹ werden Methoxy und Ethoxy besonders bevorzugt.

Als gegebenenfalls alkylsubstituierte C₃- bis C₁₂-Cycloalkylreste für R³, R⁴ und R¹⁰ kommen vorzugsweise C₅- bis C₈-Cycloalkyl, vor allem C₅- bis C₆-Cycloalkyl wie Cyclopentyl und Cyclohexyl, daneben auch Cycloheptyl, Cyclooctyl, 2- oder 3-Methylcyclopentyl, 2,3-, 2,4-, 2,5- oder 3,3-Dimethylcyclopentyl, 2-, 3- oder 4-Methylcyclohexyl, 2-, 3- oder 4-Ethylcyclohexyl und 2,4-, 3,4-, 3,5- oder 3,3-Dimethylcyclohexyl, sowie weiterhin auch Cyclopropyl, Cyclobutyl, Cyclododecyl, 3,4,5-Trimethylcyclohexyl oder 4-tert.-Butylcyclohexyl in Betracht.

Durch Alkyl- und Hydroxylgruppen substituiertes Phenyl für R³ sind beispielsweise o-, m- oder p-Tolyl, o-, m- oder p-Ethylphenyl, m- oder p-tert.-Butylphenyl, 2,4,6-Trimethylphenyl, 3,5-Di-tert.-butyl-4-hydroxyphenyl und o-, m- oder p-Hydroxyphenyl.

Als C₇- bis C₁₈-Phenylalkyl für R³, R⁴ und R¹⁰ eignen sich insbesondere 1-Phenylethyl, 2-Phenylethyl, 1-Phenylpropyl, 2-Phenylpropyl, 3-Phenylpropyl, 2-Phenyl-prop-2-yl, 4-Phenylbutyl, 2,2-Dimethyl-2-phenylethyl, 5-Phenylamyl, 10-Phenyldecyl, 12-Phenyldodecyl oder vor allem Benzyl. Als substituiertes C₇- bis C₁₈-Phenylalkyl eignen sich beispielsweise p-Methylbenzyl, 2-(p-Methylphenyl)ethyl, p-tert.-Butylbenzyl, 2-(p-tert.-Butylphenyl)ethyl und 2-(3',5'-Di-tert.-butyl-4'-hydroxyphenyl)ethyl.

C₆- bis C₁₈-Cycloalkyl-alkyl für R³ und R⁴ sind beispielsweise Cyclohexyl-methyl, 4-Methylcyclohexyl-methyl, 4-tert.-Butylcyclohexyl-methyl, 2-Cyclohexyl-ethyl, 3,3-Dimethylcyclohexyl-methyl und Cyclopentyl-methyl.

Als fünf- oder sechsgliedrige ungesättigte oder gesättigte heterocyclische Ringe für R³ mit bis zu drei Heteroatomen aus der Gruppe Stickstoff, Sauerstoff und Schwefel, welche zusätzlich benzanelliert und durch die bezeichneten Reste substituiert sein können, kommen in Betracht:

Tetrahydrofuran, Furan, Tetrahydrothiophen, Thiophen, 2,5-Dimethylthiophen, Pyrrolidin, Pyrrolin, Pyrrol, Isoxazol, Oxazol, Thiazol, Pyrazol, Imidazolin, Imdidazol, 1,2,3-Triazolidin, 1,2,3- und 1,2,4-Triazol, 1,2,3-, 1,2,4- und 1,2,5-Oxadiazol, Tetrahydropyran, Dihydropyran, 2H- und 4H-Pyran, Piperidin, 1,3- und 1,4-Dioxan, Morpholin, Pyrazan, Pyridin, α-.β- und γ-Picolin, α- und γ-Piperidon, Pyrimidin, Pyridazin, Pyrazin, 1,2,5-Oxathiazin, 1,3,5-, 1,2,3- und 1,2,4-Triazin, Benzofuran, Thionaphthen, Indolin, Indol, Isoindolin, Benzoxazol, Indazol, Benzimidazol, Chroman, Isochroman, 2H- und 4H-Chromen, Chinolin, Isochinolin, 1,2,3,4-Tetahydroisochinolin, Cinnolin, Chinazolin, Chinoxalin, Phthalazin und Benzo-1,2,3-triazin.

Das Restepaar R¹/R² darf keine primären oder sekundären Aminogruppen enthalten, da sonst das cyclische Carbonat III auch mit diesen Aminogruppen reagieren könnte und unerwünschte Nebenprodukte ergeben würde.

Beispiele für Alkoxycarbonyl-alk(en)yloxy-Gruppen für R² bei Bedeutung (b) sind Methoxycarbonyl-methoxy, 2-(Methoxycarbonyl)-ethoxy, 2-(Methoxycarbonyl)-ethenyloxy und 2-(Methoxycarbonyl)-isopropenyloxy.

Bei Bedeutung (c) sind die beiden Reste R⁶ vorzugsweise gleich oder bilden zusammen mit den beiden Sauerstoffatomen einen 1,3-Dioxan- oder 1,3-Dioxolan-Ring, welcher zusätzlich noch niedere Alkylgruppen und/oder Hydroxylalkylgruppen als Substituenten tragen kann. Beispiele solcher cyclischen Acetalgruppierungen sind 1,3-Dioxan-, 5-Methyl-1,3-dioxan-, 5, 5-Dimethyl-1,3-dioxan-, 4,5,5-Trimethyl-1,3-dioxan-, 5-Hydroxymethyl-5-methyl-1,3-dioxan-, 5,5-Bishydroxymethyl-1,3-dioxan-, 1,3-Dioxolan-, 4-Methyl-1,3-dioxolan- und 4-Hydroxymethyl-1,3-dioxolan-Gruppierungen.

Die N,N'-verbrückten Bis-tetramethylpiperidon-Verbindungen I gemäß Bedeutung (d) können auch in veretherter Form als Enolether der allgemeinen Formel 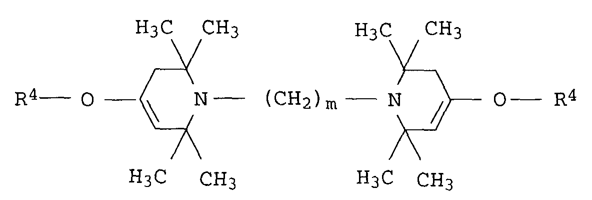 vorliegen, wobei die beiden Reste R⁴ gleich oder verschieden sind und die oben genannte Bedeutung haben.

Als Carbonsäurederivate R¹⁰-CO-R¹¹ eignen sich vor allem die entsprechenden Carbonsäurehalogenide, insbesondere Carbonsäurechloride, die Carbonsäureester, Carbonsäureanhydride sowie Kohlensäurediester, insbesondere Kohlensäuredialkylester.

Das erfindungsgemäße Verfahren zum Produkt I wird in der Regel bei Temperaturen von 60 bis 200°C, vorzugsweise 100 bis 180°C, insbesondere 140 bis 165°C durchgeführt. Beim angegebenen Temperaturbereich ist die Umsetzung meist nach 5 bis 25 Stunden vollständig abgelaufen. Da bei der Umsetzung das Gas Kohlendioxid frei wird, arbeitet man zweckmäßigerweise bei Normaldruck oder bei vermindertem Druck. Beim erfindungsgemäßen Verfahren zum Produkt Ib arbeitet man in der Regel bei den gleichen Bedingungen wie für die Herstellung des Produktes I angegeben.

Die Umsetzungen können mit oder ohne Lösungsmittel erfolgen. Als organische Lösungsmittel kommen insbesondere solche in Frage, die einen Siedepunkt über 60°C, vor allem über 100°C, insbesondere über 140°C besitzen. Als solche sind beispielsweise zu nennen:
- Alkohole wie Methanol, Ethanol, iso-Propanol, Ethylenglykol, n-Propanol, n-Butanol, iso-Butanol, tert.-Butanol und insbesondere 2-Ethylhexanol, n-Octanol oder Diethylenglykol;
- Amide wie Formamid, Dimethylformamid, Dimethylacetamid und insbesondere N-Methylpyrrolidinon;
- Aromaten wie Chlorbenzol, Toluol, Xylol, Ethylbenzol oder höher alkylierte Benzole;
- Ether wie Diisopropylether, Di-n-propylether, Diphenylether oder Tetrahydrofuran;
- tertiäre Amine wie Triethylamin, Tributylamin oder Pyridin;
- Polyethylenglykole oder Polypropylenglykole bis zu einem Molekulargewicht von ca. 1000.

Man kann auch Gemische der genannten organischen Lösungsmittel einsetzen.

In einer bevorzugten Ausführungsform verwendet man als Lösungsmittel einen Überschuß des als Reaktionspartner eingesetzten cyclischen Carbonates III. Hierbei beträgt das molare Verhältnis von Tetramethylpiperidinyl-Verbindungen II zu cyclischem Carbonat III in der Regel 1 : 0,6 bis 1 : 20, vorzugsweise 1 : 1 bis 1 : 10, insbesondere 1 : 2 bis 1 : 6, wobei für die eigentliche Umsetzung 0,5 mol cyclisches Carbonat III pro Mol Ausgangsverbindung II benötigt werden.

Ebenso ist es of vorteilhaft, bei der Umsetzung der Alkohole Ic zu den Estern Ib überschüssiges Carbonsäurederivat R¹⁰-CO-R¹¹ als Lösungsmittel zu verwenden, insbesondere, wenn mit Carbonsäureestern oder Kohlensäurediestern gearbeitet wird. Hierbei beträgt das molare Verhältnis von Alkohol Ic zu bezeichnetem Carbonsäurederivat in der Regel 1 : 2,1 bis 1 : 50, vorzugsweise 1 : 3 bis 1 : 40.

In einer weiteren bevorzugten Ausführungsform der erfindungsgemäßen Verfahren wird mit Hilfe eines Katalysators gearbeitet. Dabei wird der Katalysator in einer Menge von 0,01 bis 25 mol-%, vorzugsweise von 0,5 bis 10 mol-%, insbesondere von 1 bis 7 mol-%, bezogen auf die Menge an II bzw. Ic, eingesetzt. Eine Erhöhung der Katalysatormenge über 25 mol-% schadet der Reaktion nicht, bringt aber keine weiteren Vorteile. Die Katalysatoren kommen dabei aus folgenden Klassen:
(i) saure Katalysatoren, beispielsweise
   - Sulfonsäuren wie Methansulfonsäure, Trifluormethansulfonsäure, Benzolsulfonsäure oder p-Toluolsulfonsäure;
   - Mineralsäuren (anorganische Säuren) wie Schwefelsäure, Salzsäure oder Phosphorsäure;
   - Carbonsäuren wie Ameisen-, Essig-, Propion-, Butter-, Valerian-, Capron-, Capryl-, Caprin-, Stearin-, Öl-, Benzoe-, Methylbenzoe-, Phenylessig-, Zitronen-, Adipin-, Wein-, Nitrilotriessig- oder Ethylendiamintetraessigsäure;
(ii) schwermetallhaltige Katalysatoren, beispielsweise
   - Zinnverbindungen wie Dibutylzinnoxid, Dibutylzinndiacetat oder Dibutylzinndilaurat;
   - Titanate wie Tetramethoxytitanat, Tetra-iso-propoxytitanat oder Tetrabutoxytitanat;
(iii) organische Katalysatoren mit quaternisiertem Heteroatom, beispielsweise
   - Phosphoniumverbindungen wie die Chloride, Bromide oder Iodide der Kationen Methyltriphenylphosphonium, Ethyltriphenylphosphonium, Butyltriphenylphosphonium, Methyltributylphosphonium, Methyltriphenoxyphosphonium oder Tetrabutylphosphonium;
   - Ammoniumverbindungen wie die Chloride, Bromide, Iodide oder Hydroxide der Kationen Tetramethylammonium, Tetraethylammonium, Tetrabutylammonium, Methyltriphenylammonium, Methyltriethylammonium, Methyltributylammonium, Methyltrihexylammonium, Benzyltriethylammonium, Benzyltributylammonium, Benzyltriphenylammonium oder Benzyltrihexylammonium;
(iv) Halogenide, in der Regel in wasserfreier Form, beispielsweise
   - Alkalimetallhalogenide wie Lithiumiodid, Natriumbromid, Natriumiodid, Kaliumbromid oder Kaliumiodid;
   - Erdalkalimetallhalogenide wie Calciumchlorid, Magnesiumchlorid oder Magnesiumbromid;
   - Zinkhalogenide wie Zinkchlorid, Zinkbromid oder Zinkiodid.

Die erfindungsgemäßen Verfahren lassen sich besonders gut mit Ethylencarbonat (m = 2 in Formel III) als Komponente III durchführen.

Das erfindungsgemäße Verfahren zum Produkt I bringt auch besonders gute Resultate, wenn man von Verbindungen II ausgeht, bei denen gemäß Bedeutung (a) R¹ Wasserstoff und R² eine Gruppierung der Formel -OH, -NH-CO-R⁷ oder -O-CO-R⁷ bezeichnet, wobei R⁷ für C₁- bis C₄-Alkyl steht.

Die Erfindung wird durch die nachstehenden Beispiele weiter erläutert. Die Herstellbedingungen wurden nicht optimiert.

### Herstellungsbeispiele

### Beispiel 1

628 g (4,0 mol) 2,2,6,6-Tetramethylpiperidin-4-ol, 880 g (10,0 mol) Ethylencarbonat und 30 g (0,08 mol) Tetrabutylammoniumiodid wurden 13 h auf 155°C und weitere 6 h auf 165°C erhitzt, wobei sich CO₂ abspaltete. Man ließ abkühlen und gab bis 130°C unter Siedekühlung langsam 1 l Wasser zu. Danach ließ man unter Rühren abkühlen, saugte den entstandenen Niederschlag bei Raumtemperatur ab und wusch mit Wasser nach, bis das Filtrat farblos war. Nach dem Trocknen erhielt man 603 g der Verbindung der Formel 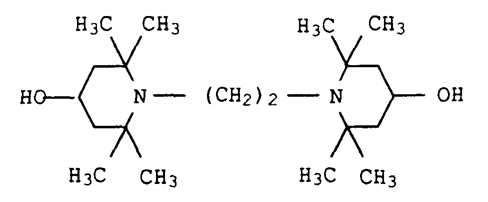 als farblosen Feststoff vom Schmp. 264-267°C.

### Beispiel 2

49,5 g (0,23 mol) 4-N-Acetyl-2,2,6,6-tetramethyl-4-aminopiperidin, 100 g (1,14 mol) Ethylencarbonat und 5 g (0,14 mol) Tetrabutylammoniumodid wurden 7 h auf 155°C erhitzt, wobei sich CO₂ abspaltete. Man ließ abkühlen, gab 150 mol Wasser zu, rührte 2 h bei 90°C und saugte den entstandenen Niederschlag bei dieser Temperatur ab. Der Rückstand wurde zweimal mit je 100 ml Ethanol gewaschen und bei 50°C im Wasserstrahlvakuum getrocknet. Man erhielt 37,6 g der Verbindung der Formel 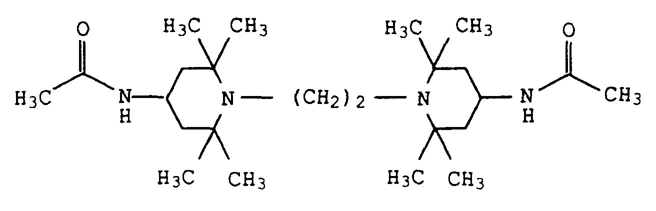 vom Schmp. >300°C.

### Beispiel 3

51 g des Produkts aus Beispiel 1, 1,5 ml Dibutylzinndiacetat und 400 g Diethylcarbonat wurden 30 h bei 125°C gerührt, dabei destillierte Ethanol ab. Man verdünnte mit Petrolether, nahm den Rückstand in 1,8 l Ethanol auf, filtrierte heiß und ließ abkühlen. Nach Abfiltrieren und Trocknen erhielt man 33,4 g der Verbindung der Formel 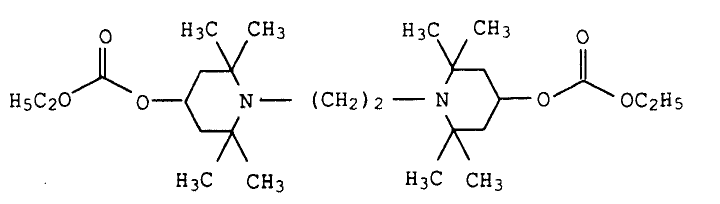 als farblosen Farbstoff vom Schmp. 187-188°C.

### Beispiel 4

In 150 ml Solvesso^{®} 100 (handelsübliches Gemisch aromatischer Kohlenwassestoffe mit einem Siedebereich von 163°C bis 170°C) wurden 34 g des Produktes aus Beispiel 1, 64,4 g 3-(3',3'-Di-tert.-butyl-4'-hydroxyphenyl)propionsäuremethylester und 1,5 ml Dibutylzinndiacetat 14 h auf 170°C erhitzt, wobei Methanol abdestillierte. Man destillierte danach das Lösungsmittel im Vakuum ab und kristallisierte den Rückstand aus Methylcyclohexan um. Nach Abfiltrieren und Trocknen erhielt man 46 g der Verbindung der Formel 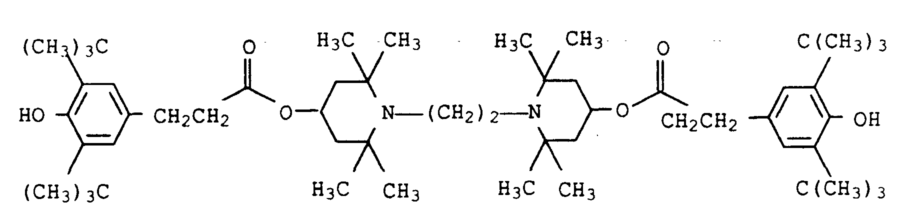 als farblosen Farbstoff vom Schmp. 207-208°C.

### Beispiel 5

### Synthese der Verbindung aus Beispiel 1 mit verschiedenen Katalysatoren unter standardisierten Bedingungen

31,4 g 2,2,6,6-Tetramethylpiperidin-4-ol, 44 g Ethylencarbonat und 0,5 g Katalysator gemäß Tabelle 1 wurden 7 h auf 150-155°C erhitzt. Anschließend wurde auf 90°C abgekühlt und 150 ml Wasser wurden zugetropft. Es wurde 0,5 h unter Rückfluß erhitzt und der entstandene Niederschlag wurde bei 95°C abfiltriert. Das Produkt wurde mit 50 ml Wasser von 90°C gewaschen und im Wasserstrahlvakuum bei 110°C getrocknet.

Die erhaltenen Ausbeuten an Produkt sind in der Tabelle zusammengestellt.

**Tabelle**

| | |
|---|---|
| Umsetzung von 2,2,6,6-Tetramethylpiperidin-4-ol mit Ethylencarbonat mit verschiedenen Katalysatoren | |

| Katalysator | Ausbeute [%] |
|---|---|
| Lithiumiodid | 89,4 |
| Natriumbromid | 83,2 |
| Natriumiodid | 85,3 |
| Kaliumbromid | 64,1 |
| Kaliumiodid | 60,2 |
| Magnesiumchlorid | 80,0 |
| Magnesiumbromid | 90,9 |
| Tetrabutylammoniumbromid | 55,3 |
| Tetrabutylammoniumiodid | 57,9 |
| Tetraethylammoniumiodid | 50,9 |
| Tetramethylammoniumiodid | 60,3 |
| Methyltriphenylphosphoniumiodid | 75,3 |
| Methyltriphenoxyphosphoniumiodid | 83,5 |

## Patentansprüche

1. Verfahren zur Herstellung von N,N'-verbrückten Bis-tetramethylpiperidinyl-Verbindungen der allgemeinen Formel I 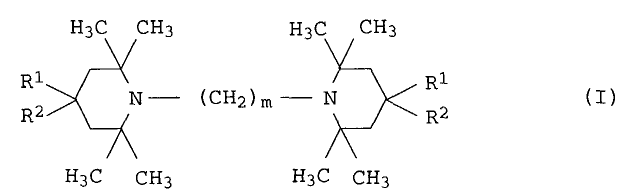 in der die Variable m für die Zahl 2 oder 3 steht und die beiden Restepaare R¹/R² gleich oder verschieden sind und folgende Bedeutungen haben können:
(a)
R¹ Wasserstoff und R² eine Gruppierung der Formel -A-R³, wobei
A Sauerstoff oder ein Brückenglied der Formel -NR⁴-, -NR⁵-CO-, -O-CO-, -O-CO-NR⁵- oder -O-CO-O- bedeutet,
R³ Wasserstoff, C₁- bis C₂₀-Alkyl, das durch bis zu 10 nicht benachbarte Sauerstoffatome oder Brückenglieder der Formel -NR⁴- und durch bis zu 3 Carbonylgruppen unterbrochen sein kann, C₂- bis C₂₀-Alkenyl, C₃- bis C₁₂-Cycloalkyl, das durch bis zu 3 C₁- bis C₄-Alkylgruppen substituiert sein kann, Phenyl, das durch bis zu 3 C₁- bis C₆-Alkylgruppen oder Hydroxylgruppen substituiert sein kann, C₇- bis C₁₈-Phenylalkyl, das durch bis zu 3 C₁- bis C₄-Alkylgruppen oder Hydroxylgruppen substituiert sein kann, C₆- bis C₁₈-Cycloalkyl-alkyl, das durch bis zu 3 C₁- bis C₄-Alkylgruppen substituiert sein kann, oder einen fünf- oder sechsgliedrigen ungesättigten oder gesättigten heterocyclischen Ring mit bis zu 3 Heteroatomen aus der Gruppe Stickstoff, Sauerstoff und Schwefel, welcher zusätzlich benzanelliert und durch bis zu 3 C₁- bis C₆-Alkylgruppen oder Hydroxylgruppen substituiert sein kann, bezeichnet, mit der Maßgabe, daß R³ nicht Wasserstoff bezeichnet, falls A das Brückenglied -NR⁴- bedeutet,
R⁴ C₁- bis C₂₀-Alkyl, C₂- bis C₂₀-Alkenyl, C₃- bis C₁₂-Cycloalkyl, das durch bis zu 3 C₁- bis C₄-Alkylgruppen substituiert sein kann, C₇ - bis C₁₈-Phenylalkyl, das durch bis zu 3 C₁- bis C₄-Alkylgruppen substituiert sein kann, oder C₆- bis C₁₈-Cycloalkylalkyl, das durch bis zu 3 C₁- bis C₄-Alkylgruppen substituiert sein kann, bedeutet und
R⁵ Wasserstoff oder C₁- bis C₄-Alkyl bezeichnet;
(b)
R¹ Wasserstoff und R² C₁- bis C₆-Alkoxycarbonyl-C₁- bis C₂₀-alkyloxy oder C₁- bis C₆-Alkoxycarbonyl-C₂- bis C₂₀-alkenyloxy;
(c)
R¹ und R² Gruppierungen der Formel -O-R⁶, wobei die beiden Reste R⁶ jeweils gleich oder verschieden sind, für C₁- bis C₂₀-Alkyl oder C₂- bis C₂₀-Alkenyl, welches durch bis zu 3 Hydroxylgruppen substituiert sein kann, stehen und unter Ausbildung eines 1,3-Dioxan- oder eines 1,3-Dioxolan-Ringes verknüpft sein können;
(d)
R¹ / R² das Carbonyl-Sauerstoffatom eines Piperidon-Ringes, wobei diese Sauerstoffatome auch unter Ausbildung der entsprechenden Enol-Struktur am Piperidon-Ring durch Gruppierungen der Struktur R⁴ verethert sein können;
ausgehend von Tetramethylpiperidinyl-Verbindungen der allgemeinen Formel II 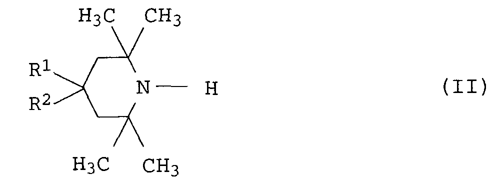 dadurch gekennzeichnet, daß man die Verbindungen II mit einem cyclischen Carbonat der allgemeinen Formel III 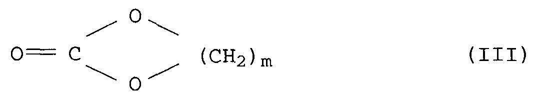 umsetzt.

2. Verfahren zur Herstellung von N,N'-verbrückten Bis-tetramethylpiperidinyl-Verbindungen I nach Anspruch 1, dadurch gekennzeichnet, daß man als Lösungsmittel einen Überschuß des cyclischen Carbonates III einsetzt, wobei das molare Verhältnis von II zu III 1 : 0,6 bis 1 : 20 beträgt.

3. Verfahren zur Herstellung von N,N'-verbrückten Bis-tetramethylpiperidinyl-Verbindungen I nach Anspruch 1 oder 2, dadurch gekennzeichnet, daß man die Umsetzung in Gegenwart von 0,01 bis 25 mol-%, bezogen auf die Menge an eingesetzten Tetramethylpiperidinyl-Verbindungen II, eines sauren Katalysators, eines schwermetallhaltigen Katalysators, eines organischen Katalysators mit quaternisiertem Heteroatom oder eines Halogenids als Katalysator durchführt.

4. Verfahren zur Herstellung von N,N'-verbrückten Bis-tetramethylpiperidinyl-Verbindungen I nach den Ansprüchen 1 bis 3, dadurch gekennzeichnet, daß man als cyclisches Carbonat III Ethylencarbonat einsetzt.

5. Verfahren zur Herstellung von N,N'-verbrückten Bis-tetramethylpiperidinyl-Verbindungen I nach den Ansprüchen 1 bis 4, ausgehend von Verbindungen II, bei denen gemäß Bedeutung (a) R¹ Wasserstoff und R² eine Gruppierung der Formel -OH, -NH-CO-R⁷ oder -O-CO-R⁷ bezeichnet, wobei R⁷ für C₁- bis C₄-Alkyl steht.

6. Verfahren zur Herstellung von N,N'-verbrückten Bis-tetramethylpiperidinylestern der allgemeinen Formel Ib 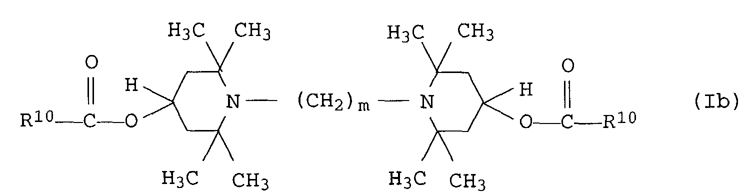 in der die Variable m für die Zahl 2 oder 3 steht und R¹⁰ C₁- bis C₂₀-Alkyl oder C₂- bis C₂₀-Alkenyl, welches durch bis zu 3 Hydroxylgruppen substituiert sein kann, C₁- bis C₂₀-Alkoxy, C₃- bis C₁₂-Cycloalkyl, das durch bis zu 3 C₁- bis C₄-Alkylgruppen substituiert sein kann, Phenyl, das durch bis zu 3 C₁- bis C₆-Alkylgruppen oder Hydroxylgruppen substituiert sein kann, oder C₇- bis C₁₈-Phenylalkyl, das durch bis zu 3 C₁- bis C₄-Alkylgruppen oder Hydroxylgruppen substituiert sein kann, bedeutet, dadurch gekennzeichnet, daß man nach dem Verfahren gemäß Anspruch 1 hergestellte N,N'-verbrückte Bistetramethyl-hydroxypiperidinyl-Verbindungen der Formel Ic 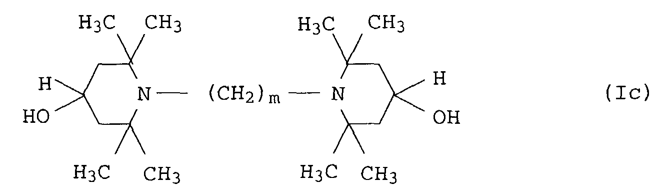 mit Carbonsäurederivaten der allgemeinen Formel R¹⁰-CO-R¹¹ umsetzt, in der R¹¹ Chlor, Brom, C₁- bis C₄-Alkoxy oder eine Gruppe der Formel -O-CO-R¹⁰ bezeichnet.

7. Verfahren zur Herstellung von N,N'-verbrückten Bis-tetramethylpiperidinylestern Ib nach Anspruch 6, dadurch gekennzeichnet, daß man als Lösungsmittel einen Überschuß des als Reaktionspartner verwendeten Carbonsäurederivates einsetzt, wobei das molare Verhältnis von Ic zu Carbonsäurederivat 1 : 2,1 bis 1 : 50 beträgt.

8. Verfahren zur Herstellung von N,N'-verbrückten Bis-tetramethylpiperidinylestern Ib nach Anspruch 6 oder 7, dadurch gekennzeichnet, daß man die Umsetzung in Gegenwart von 0,01 bis 25 mol-%, bezogen auf die Menge an eingesetztem Alkohol Ic, eines sauren Katalysators, eines schwermetallhaltigen Katalysators, eines organischen Katalysators mit quaternisiertem Heteroatom oder eines Halogenids als Katalysator durchführt.

## Claims

1. A process for the preparation of N,N'-bridged bistetramethylpiperidinyl compounds of the formula I 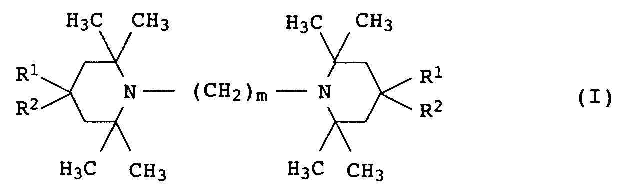 where m is 2 or 3 and the two pairs of radicals R¹/R² are identical or different and may have the following meanings:
(a)
R¹ is hydrogen and R² is a group of the formula -A-R³,
A is oxygen or a bridging member of the formula -NR⁴-, -NR⁵-CO-, -O-CO-, -O-CO-NR⁵- or O-CO-O-,
R³ is hydrogen or C₁-C₂₀-alkyl which may be interrupted by up to 10 non-neighboring oxygen atoms or bridging members of the formula -NR⁴- and by up to 3 carbonyl groups, C₂-C₂₀-alkenyl, C₃-C₁₂-cycloalkyl which may be substituted by up to 3 C₁-C₄-alkyl groups, phenyl which may be substituted by up to 3 C₁-C₆-alkyl groups or hydroxyl groups, C₇-C₁₈-phenylalkyl which may be substituted by up to 3 C₁-C₄-alkyl groups or hydroxyl groups, C₆-C₁₈-cycloalkylalkyl which may be substituted by up to 3 C₁-C₄-alkyl groups, or a five-membered or six-membered unsaturated or saturated heterocyclic ring having up to 3 hetero atoms selected from the group consisting of nitrogen, oxygen and sulfur, which may additionally be benzofused and substituted by up to 3 C₁-C₆-alkyl groups or hydroxyl groups, with the proviso that R³ is not hydrogen if A is the bridging member -NR⁴-,
R⁴ is C₁-C₂₀-alkyl, C₂-C₂₀-alkenyl, C₃-C₁₂-cycloalkyl which may be substituted by up to 3 C₁-C₄-alkyl groups, C₇-C₁₈-phenylalkyl which may be substituted by up to 3 C₁-C₄-alkyl groups, or C₆-C₁₈-cycloalkylalkyl which may be substituted by up to 3 C₁-C₄-alkyl groups and
R⁵ is hydrogen or C₁-C₄-alkyl;
(b)
R¹ is hydrogen and R² is C₁-C₆-alkoxycarbonyl- or C₁-C₆-alkoxycarbonyl-C₂-C₂₀-alkenyloxy;
(c)
R¹ and R² are each a group of the formula -O-R⁶, where the two radicals R⁶ are identical or different and are each C₁-C₂₀-alkyl or C₂-C₂₀-alkenyl, which may be substituted by up to 3 hydroxyl groups, and may be linked to form a 1,3-dioxane or a 1,3-dioxolane ring;
(d)
R¹ / R² is the carbonyl oxygen atom of a piperidone ring, where these oxygen atoms may furthermore be etherified by groups of the structure R⁴ to form the corresponding enol structure on the piperidone ring;
starting from tetramethylpiperidinyl compounds of the formula II 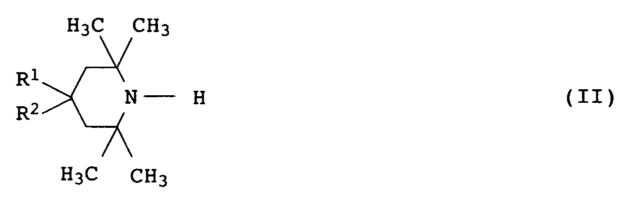 which comprises reacting a compound II with a cyclic carbonate of the formula III

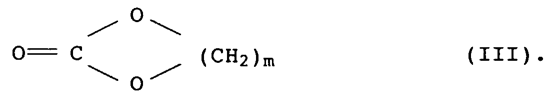

2. A process for the preparation of N,N'-bridged bistetramethylpiperidinyl compounds I as claimed in claim 1, wherein an excess of the cyclic carbonate III is used as the solvent, the molar ratio of II to III being from 1:0.6 to 1:20.

3. A process for the preparation of N,N'-bridged bistetramethylpiperidinyl compounds I as claimed in claim 1 or 2, wherein the reaction is carried out in the presence of from 0.01 to 25 mol%, based on the amount of tetramethylpiperidinyl compound II used, of an acidic catalyst, of a heavy-metal-containing catalyst, of an organic catalyst having a quaternized hetero atom or of a halide as the catalyst.

4. A process for the preparation of N,N'-bridged bistetramethylpiperidinyl compounds I as claimed in any of claims 1 to 3, wherein ethylene carbonate is used as the cyclic carbonate III.

5. A process for the preparation of N,N'-bridged bistetramethylpiperidinyl compounds I as claimed in any of claims 1 to 4, starting from a compound II in which, according to the definition under (a), R¹ is hydrogen and R² is a group of the formula -OH, -NH-CO-R⁷ or -O-CO-R⁷, R⁷ being C₁-C₄-alkyl.

6. A process for the preparation of N,N'-bridged bistetramethylpiperidinyl esters of the formula Ib 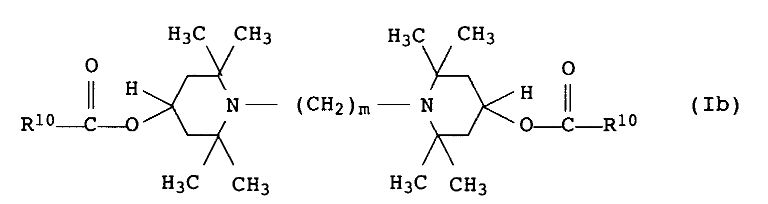 where m is 2 or 3 and R¹⁰ is C₁-C₂₀-alkyl or C₂-C₂₀-alkenyl, which may be substituted by up to 3 hydroxyl groups, C₁-C₂₀-alkoxy, C₃-C₁₂-cycloalkyl, which may be substituted by up to 3 C₁-C₄-alkyl groups, phenyl which may be substituted by up to 3 C₁-C₆-alkyl groups or hydroxyl groups, or C₇-C₁₈-phenylalkyl which may be substituted by up to 3 C₁-C₄-alkyl groups or hydroxyl groups, wherein an N,N'-bridged bistetramethylhydroxypiperidinyl compound of the formula Ic 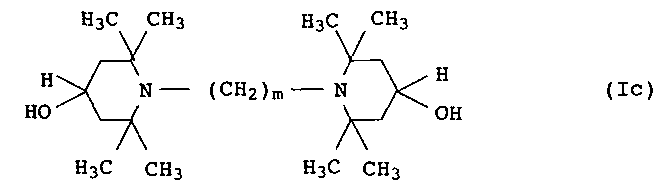 prepared by the process as claimed in claim 1 is reacted with a carboxylic acid derivative of the formula R¹⁰-CO-R¹¹, where R¹¹ is chlorine, bromine, C₁-C₄-alkoxy or a group of the formula -O-CO-R¹⁰.

7. A process for the preparation of N,N'-bridged bistetramethylpiperidinyl esters Ib as claimed in claim 6, wherein an excess of the carboxylic acid derivative used as a reactant is employed as the solvent, the molar ratio of Ic to carboxylic acid derivative being from 1:2.1 to 1:50.

8. A process for the preparation of N,N'-bridged bistetramethylpiperidinyl esters Ib as claimed in claim 6 or 7, wherein the reaction is carried out in the presence of from 0.01 to 25 mol%, based on the amount of alcohol Ic used, of an acidic catalyst, of a heavy-metal-containing catalyst, of an organic catalyst having a quaternized hetero atom or of a halide as the catalyst.

## Revendications

1. Procédé de préparation de composés bis-tétraméthylpipéridinyle N,N'-pontés de formule générale I 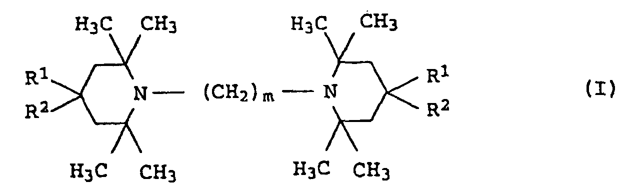 dans laquelle la variable m est mise pour le nombre 2 ou 3, et les deux paires de restes R¹/R² sont identiques ou différentes et peuvent avoir les significations suivantes:
(a)
R¹ représente un atome d'hydrogène et R² un groupement de formule -A-R³, où
A représente un atome d'oxygène ou un maillon pontant de formule -NR⁴-, -NR⁵-CO-, -O-CO-, -O-CO-NR⁵- ou -O-CO-O-
R³ représente un atome d'hydrogène, un groupement alkyle en C₁-C₂₀, pouvant être interrompu par jusqu'à 10 atomes d'oxygène non voisins ou maillons pontants de formule -NR⁴- et par jusqu'à 3 groupements carbonyle, un groupement alcényle en C₂-C₂₀, cycloalkyle en C₃-C₁₂, pouvant être substitué par jusqu'à 3 groupements alkyle en C₁-C₄, un groupement phényle, pouvant être substitué par jusqu'à 3 groupements alkyle en C₁-C₆, ou groupements hydroxyle, un groupement phénylalkyle en C₇-C₁₈, pouvant être substitué par jusqu'à 3 groupements alkyle en C₁-C₄ ou hydroxyle, un groupement (cycloalkyle en C₆-C₁₈)alkyle, pouvant être substitué par jusqu'à 3 groupements alkyle en C₁-C₄, ou bien un cycle hétérocyclique saturé ou insaturé à 5 ou 6 maillons ayant jusqu'à 3 hétéroatomes choisis dans le groupe de l'azote, de l'oxygène et du soufre, lequel peut en outre être benzocondensé et substitué par jusqu'à 3 groupements alkyle en C₁-C₆, ou hydroxyle, étant spécifié que R³ ne représente pas de l'hydrogène lorsque A représente le maillon pontant -NR⁴-,
R⁴ représente un groupement alkyle en C₁-C₂₀, alcényle en C₂-C₂₀, cycloalkyle en C₃-C₁₂, pouvant être substitué par jusqu'à 3 groupements alkyle en C₁-C₄, un groupement (phényle en C₇-C₁₈)alkyle pouvant être substitué par jusqu'à 3 groupements alkyle en C₁-C₄, ou un groupement cycloalkyle en C₆-C₁₈, pouvant être substitué par jusqu'à 3 groupements alkyle en C₁-C₄, et
R⁵ représente un atome d'hydrogène ou un groupement alkyle en C₁-C₄,
(b)
R¹ représente un atome d'hydrogène et R² représente un groupement (alcoxycarbonyle en C₁-C₆)(alkyloxy en C₁-C₂₀) ou un groupement (alcoxycarbonyle en C₁-C₆)(alcényloxy en C₂-C₂₀);
(c)
R¹ et R² représentent des groupements de formule -O-R⁶, où les deux restes R⁶ sont identiques ou différents, et sont mis pour des groupements alkyle en C₁-C₂₀ ou alcényle en C₂-C₂₀, pouvant être substitué par jusqu'à 3 groupements hydroxyle, et pouvant être reliés avec formation d'un cycle 1,3-dioxane ou 1,3-dioxolane;
(d)
R¹ / R² représentent l'atome d'oxygène du carbonyle d'un cycle pipéridone, où ces atomes d'oxygène peuvent également être éthérifiés par des groupements de structure R⁴ avec formation de la structure énol correspondante sur le cycle pipéridone;
à partir de composés tétraméthylpipéridinyle de formule générale II 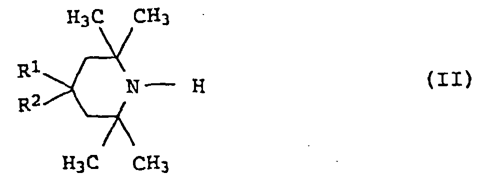 caractérisé en ce que l'on fait réagir les composés II avec un carbonate cyclique de formule générale III

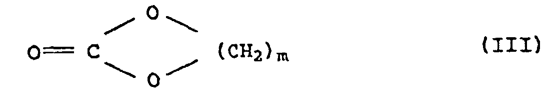

2. Procédé de préparation de composés bis-tétraméthylpipéridinyle N,N'-pontés I selon la revendication 1, caractérisé en ce que l'on utilise en tant que solvant un excès du carbonate cyclique III, où le rapport molaire de Il à III vaut de 1:0,6 à 1:20.

3. Procédé de préparation de composés bis-tétraméthylpipéridinyle N,N'-pontés I selon la revendication 1 ou 2, caractérisé en ce que l'on met en oeuvre la réaction en présence de 0,01 à 25% en moles, par rapport à la quantité de composés II tétraméthylpipéridinyle utilisée, d'un catalyseur acide, d'un catalyseur contenant des métaux lourds, d'un catalyseur organique ayant un hétéroatome quaternisé ou d'un halogénure en tant que catalyseur.

4. Procédé de préparation de composés bis-tétraméthylpipéridinyle N,N'-pontés I selon l'une quelconque des revendications 1 à 3, caractérisé en ce que l'on utilise du carbonate d'éthylène en tant que carbonate cyclique III.

5. Procédé de préparation de composés bis-tétraméthylpipéridinyle N,N'-pontés I selon l'une quelconque des revendications 1 à 4, à partir de composés II, pour lequels selon la signification (a), R¹ représente un atome d'hydrogène et R² un groupement de formule -OH, -NH-CO-R⁷ ou -O-CO-R⁷, où R⁷ est mis pour un groupement alkyle en C₁-C₄.

6. Procédé de préparation de composés bis-tétraméthylpipéridinylesters N,N'-pontés I de formule générale Ib 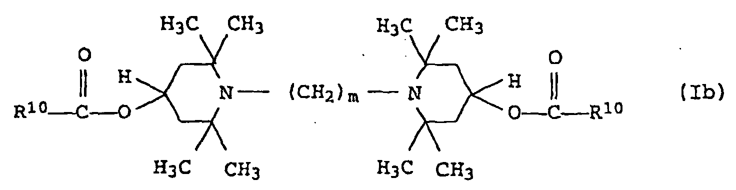 dans laquelle la variable m est mise pour le nombre 2 ou 3 et R¹⁰ représente un groupement alkyle en C₁-C₂₀ ou alcényle en C₂-C₂₀, pouvant être substitué par jusqu'à 3 groupements hydroxyle, un groupement alcoxy en C₁-C₂₀, un groupement cycloalkyle en C₃-C₁₂, pouvant être substitué par jusqu'à 3 groupements alkyle en C₁-C₄, un groupement phényle, pouvant être substitué par jusqu'à 3 groupements alkyle en C₁-C₆, ou groupements hydroxyle, ou un groupement phénylalkyle en C₇-C₁₈, pouvant être substitué par jusqu'à 3 groupements alkyle en C₁-C₄ ou hydroxyle, caractérisé en ce que l'on fait réagir des composés bis-tétraméthylhydroxypipéridinyle N,N'-pontés I de formule lc 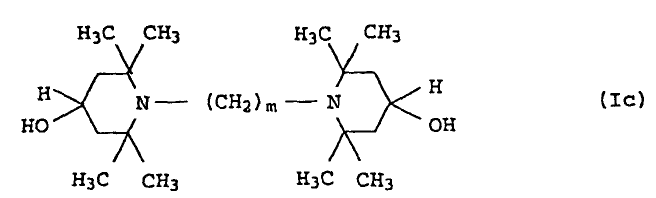 obtenus par le procédé selon la revendication 1, avec des dérivés d'acide carboxylique de formule générale R¹⁰-CO-R¹¹, dans laquelle R¹¹ représente du chlore, du brome, un groupement alcoxy en C₁-C₄ ou un groupement de formule -O-CO-R¹⁰.

7. Procédé de préparation de composés bis-tétraméthylpipéridinylesters N,N'-pontés Ib selon la revendication 6, caractérisé en ce que l'on utilise en tant que solvant un excès du dérivé d'acide carboxylique utilisé comme partenaire réactionnel, où le rapport molaire de Ic au dérivé d'acide carboxylique vaut de 1:2,1 à 1:50.

8. Procédé de préparation de composés bis-tétraméthylpipéridinylesters N,N'-pontés Ib selon la revendication 6 ou 7, caractérisé en ce que l'on met en oeuvre la réaction en présence de 0,01 à 25% en moles, par rapport à la quantité d'alcool Ic utilisée, d'un catalyseur acide, d'un catalyseur contenant des métaux lourds, d'un catalyseur organique ayant un hétéroatome quaternisé ou d'un halogénure en tant que catalyseur.
